# EUROPÄISCHE PATENTANMELDUNG

(11) **EP 0 697 192 A1**
(43) Veröffentlichungstag der Anmeldung: **21.02.1996**
(21) Anmeldenummer: 95202168.1
(22) Anmeldetag: 09.08.1995
(51) Int. Cl.: A61B 6/00

(54) **Anordnung zur Erzeugung von Röntgenaufnahmen**

(30) Priorität: 13.08.1994 DE 4428779
(71) Anmelder: Philips Patentverwaltung GmbH, D-22335 Hamburg (DE); PHILIPS ELECTRONICS N.V., NL-5621 BA Eindhoven (NL)
(72) Erfinder: Oldendorf, Frank, c/o Philips, D-20097 Hamburg (DE); Haarmann, Heinz, c/o Philips, D-20097 Hamburg (DE); Libera, Anja, c/o Philips, D-20097 Hamburg (DE); Spitzmann, Gerd, c/o Philips, D-20097 Hamburg (DE)
(74) Vertreter: Hartmann, Heinrich, Dipl.-Ing.

(57) **Zusammenfassung**

Die Erfindung betrifft eine Anordnung zur Erzeugung von Röntgenaufnahmen mit einem Röntgenstrahler (1) zur Erzeugung eines Röntgenstrahlenbündels (11), einem Photoleiter (7) zur Umsetzung von Röntgenstrahlung in ein Ladungsmuster, der auf einem rotationssymmetrisch ausgebildeten drehbaren Träger (6) aufgebracht ist, einer Aufladeeinrichtung (16) zur homogenen Aufladung der Oberfläche des rotierenden Photoleiters (7) vor der Röntgenaufnahme, einer Ausleseeinheit (17), um nach einer Röntgenaufnahme das Ladungsmuster auf der Oberfläche des rotierenden Photoleiters (7) in elektrische Bildwerte umzusetzen und einem Gehäuse (5) zur Lagerung der rotierenden Teile, das einen für Röntgenstrahlung transparenten Bereich (9,10) aufweist.

Bei der Erfindung werden Röntgenaufnahmen eines Patienten in mindestens einer weiteren Stellung außer der liegenden dadurch ermöglicht, daß der Röntgenstrahler (1) in einem Verstellwinkelbereich um die Drehachse (12) in verschiedenen Winkelstellungen positionierbar ist und daß sich der für Röntgenstrahlung transparente Bereich (9,10) über einen solchen Bereich des Gehäuses (5) erstreckt, daß eine Belichtung des Photoleiters (7) aus jeder der im Verstellwinkelbereich einstellbaren Winkelpositionen des Röntgenstrahlers (1) möglich ist.

## Beschreibung

Die Erfindung bezieht sich auf eine Anordnung zur Erzeugung von Röntgenaufnahmen mit einem Röntgenstrahler zur Erzeugung eines Röntgenstrahlenbündels, einem Photoleiter zur Umsetzung von Röntgenstrahlung in ein Ladungsmuster, der auf einem rotationssymmetrisch ausgebildeten drehbaren Träger aufgebracht ist, einer Aufladeeinrichtung zur homogenen Aufladung der Oberfläche des rotierenden Photoleiters vor der Röntgenaufnahme, einer Ausleseeinheit, um nach einer Röntgenaufnahme das Ladungsmuster auf der Oberfläche des rotierenden Photoleiters in elektrische Bildwerte umzusetzen und einem Gehäuse zur Lagerung der rotierenden Teile, das einen für Röntgenstrahlung transparenten Bereich aufweist.

Eine solche Anordnung ist aus der DE-A 40 15 113 bekannt. Dabei erfolgt die Belichtung während der Aufnahme von einem senkrecht über dem Photoleiter angeordneten Röntgenstrahler aus. Der Patient liegt auf einer Tischplatte oberhalb des Photoleiters. Die Tischplatte und der darunter befindliche, im Strahlengang liegende Teil des den rotierenden Photoleiter umgebenden Gehäuses bestehen aus für Röntgenstrahlung transparentem Material.

Der vorliegenden Erfindung liegt die Aufgabe zugrunde, eine Anordnung zu schaffen, die es ermöglicht, von einem Patienten außer in der liegenden in mindestens einer weiteren Stellung Röntgenaufnahmen zu machen.

Ausgehend von einer Anordnung der eingangs genannten Art wird diese Aufgabe erfindungsgemäß dadurch gelöst, daß der Röntgenstrahler in einem Verstellwinkelbereich um die Drehachse in verschiedenen Winkelstellungen positionierbar ist und daß sich der für Röntgenstrahlung transparente Bereich über einen solchen Bereich des Gehäuses erstreckt, daß eine Belichtung des Photoleiters aus jeder der im Verstellwinkelbereich einstellbaren Winkelpositionen des Röntgenstrahlers möglich ist.

Während bei der bekannten Anordnung nur eine einzige Strahlungsrichtung möglich ist, kann hier die Strahlungsrichtung in einem Verstellwinkelbereich um die Drehachse variiert werden. Als Vorteil ergibt sich daraus, daß der Patient nicht nur im Liegen von oben sondern auch in anderen Stellungen wie beispielsweise im Stehen von der Seite durchstrahlt werden kann. Bei feststehend senkrecht verlaufender Drehachse ist diese gewünschte Variation der Aufnahmerichtung nicht möglich. Die Drehachse des Trägers an die gewünschte Aufnahmerichtung anzupassen, d.h. bei Röntgenaufnahmen von oben die Drehachse horizontal, bei Röntgenaufnahmen von der Seite vertikal auszurichten, führt zu einer Lösung der gestellten Aufgabe. Vorteilhafter ist eine Ausgestaltung der Erfindung, bei der die Drehachse des rotationssymmetrisch ausgebildeten Trägers in horizontaler Richtung verläuft. Eine Vorrichtung zum Schwenken des Trägers zur Anpassung seiner Drehachse kann hier entfallen.

Eine bevorzugte Weiterbildung sieht vor, daß der Verstellwinkelbereich mindestens 90° beträgt. Vom Patienten können dadurch die am häufigsten benötigten Röntgenaufnahmen direkt senkrecht von oben in liegender Stellung und in horizontaler Richtung in stehender Stellung gemacht werden. So kann nacheinander an einem Gerät beispielsweise eine Röntgenaufnahme der Wirbelsäule in belastetem und in unbelastetem Zustand erfolgen.

In weiterer Ausgestaltung der Erfindung ist vorgesehen, daß die Vorrichtung zum Aufladen der Oberfläche des Photoleiters vor der Belichtung und die Einheit zum Auslesen der Röntgenaufnahme so angeordnet sind, daß sie in jeder der möglichen Winkelpositionen des Röntgenstrahlers außerhalb des Strahlengangs zwischen Röntgenstrahler und Photoleiter liegen.

Eine weitere Ausgestaltung sieht vor, daß das Gehäuse, welches den rotationssymmetrisch ausgebildeten Träger umgibt und in welchem dieser gelagert ist, aus zueinander im wesentlichen rechtwinklig angeordneten ebenen Wandungen besteht, was eine einfache Bauweise erlaubt. Zwei einander benachbarte Wandungen, vorzugsweise die obere waagrechte und eine seitliche Wandung bestehen aus für Röntgenstrahlung transparentem Material.

In einer weiteren Ausgestaltung sind mindestens zwei an sich bekannte Sensoreinrichtungen zur Erfassung der sich auf der Oberfläche des Photoleiters während der Belichtung aufbauenden Ladungsstärke vorgesehen. Diese Sensoreinrichtungen sind derart angeordnet, daß jeweils eine für eine bestimmte Bestrahlungsrichtung im Strahlengang zwischen Röntgenstrahler und Photoleiter liegt. Eine in einer weiteren Ausgestaltung vorgesehene Steuerung schaltet bei ausreichender Belichtung des Photoleiters, was durch eine der Sensoreinrichtungen überwacht wird, die Röntgenstrahlung automatisch ab. Für den Patienten bedeutet dies, daß er nur mit der für eine bestimmte gewünschte Bildauflösung erforderlichen Röntgenstrahlungsdosis belastet wird. Eine in einer weiteren Ausgestaltung vorgesehene Steuerung wählt aus den vorhandenen Sensoreinrichtungen abhängig von der Winkelposition des Röntgenstrahlers vor der Belichtung die zugehörige, im Strahlengang liegende Sensoreinrichtung aus und aktiviert diese. Dadurch wird dem Bedienpersonal diese Aufgabe abgenommen und verhindert somit auch eine Fehlbedienung durch falsche oder keine Wahl einer Sensoreinrichtung, was wiederum dem Schutz des Patienten dient.

Eine bevorzugte Ausgestaltung sieht Mittel zur Höhenverstellung des Gehäuses vor, um dessen Höhenlage der Größe und/oder der Stellung des Patienten anpassen zu können. Weiterhin kann eine Steuerung vorgesehen sein, die abhängig von der Höhe des Gehäuses und der gewünschten Strahlungsrichtung automatisch den Röntgenstrahler in die zugeordnete Position bringt. Dies ermöglicht eine einfache Bedienung der gesamten Anordnung und verringert die Wahrscheinlichkeit einer Fehlbelichtung beispielsweise durch falsche Einstellung der Strahlungsrichtung oder durch falschen Abstand des Röntgenstrahlers vom Photoleiter.

Die Erfindung wird nachstehend anhand der Zeichnungen näher erläutert. Es zeigen
**Fig. 1** ein Röntgengerät nach der Erfindung in schematischer Darstellung,
**Fig. 2** eine Anordnung zur Röntgenaufnahme eines liegenden Patienten von oben mit senkrechter Strahlungsrichtung,
**Fig. 3** eine Anordnung zur Röntgenaufnahme eines stehenden Patienten von der Seite mit horizontaler Strahlungsrichtung,
**Fig. 4** eine Anordnung zur Röntgenaufnahme eines auf einem zusätlichen Tisch liegenden Patienten von der Seite mit horizontaler Strahlungsrichtung,
**Fig. 5** einen Schnitt durch das Gehäuse mit einer zweckmäßigen Anordnung von Auflade- und Ausleseeinheit sowie der Sensoreinrichtungen.

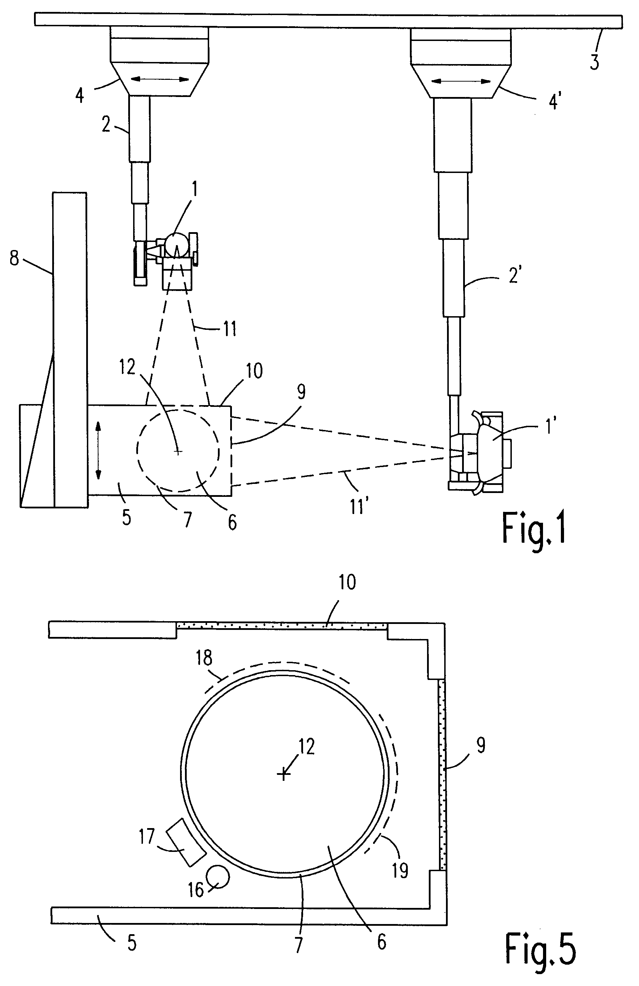

In Fig. 1 ist mit 1 eine Röntgenstrahlenquelle bezeichnet, die in beliebiger Richtung schwenkbar an einem in seiner Länge variablen vertikalen Teleskoparm 2 befestigt ist. Über eine an der Decke befestigte Schiene 3 kann der an einem Schienenläufer 4 angebrachte Teleskoparm 2 in horizontaler Richtung positioniert werden. Die mit 1, 2 und 4 bezeichneten Bauteile sind in der rechts dargestellten Position für horizontale Strahlungsrichtung mit 1', 2' und 4' bezeichnet. Das Gehäuse 5, in dem der rotationssymmetrisch ausgebildete Träger 6 gelagert ist, auf dem der Photoleiter 7 aufgebracht ist, ist mit einem Bodenstativ 8 verbunden. Die Drehachse 12, um die der Träger 6 mittels einer nicht dargestellten Antriebsvorrichtung drehbar ist, steht senkrecht zur Zeichenebene und verläuft in horizontaler Richtung. Das Gehäuse 5 wird aus zueinander senkrecht angeordneten ebenen Wandungen gebildet, von denen zwei aneinander stoßende Wandungen, vorteilhafterweise die dem Bodenstativ 8 gegenüberliegende Seitenwandung 9 und die obere waagrechte Wandung 10 mindestens in dem Bereich aus für Röntgenstrahlung transparentem aber für sichtbares Licht nicht transparentem Material, beispielsweise Aluminium, bestehen, der zwischen dem Röntgenstrahler 1 und dem Photoleiter 7 im Strahlenbündel 11 bei jeder der möglichen Positionen des Röntgenstrahlers 1 liegen kann.

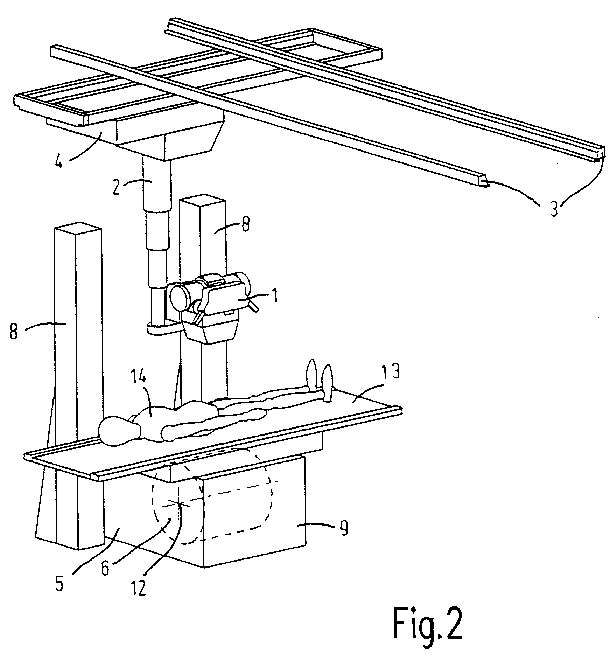

Die Figuren 2 bis 4 zeigen in perspektivischer Darstellung Anordnungen für Röntgenaufnahmen bei verschiedener Lage eines Patienten.

In Fig. 2 ist die Anordnung zur Aufnahme eines liegenden Patienten 14 dargestellt. Um es dem Patienten 14 zu erleichtern, sich auf die Tischplatte 13 zu legen bzw. um es dem Personal zu erleichtern, den Patienten 14 auf die Tischplatte 13 zu legen, kann das Gehäuse 5 mit der darauf befestigten Tischplatte 13 an dem Bodenstativ 8 bis in die durch den Durchmesser des zylinderförmigen Trägers 6 begrenzte niedrigste Position gebracht werden. Um das Körperteil, von dem eine Röntgenaufnahme gemacht werden soll, in den Strahlengang 11 zu bringen, kann die Tischplatte 13 in der parallel zur Drehachse 12 verlaufenden Richtung in der horizontalen Ebene verschoben werden.

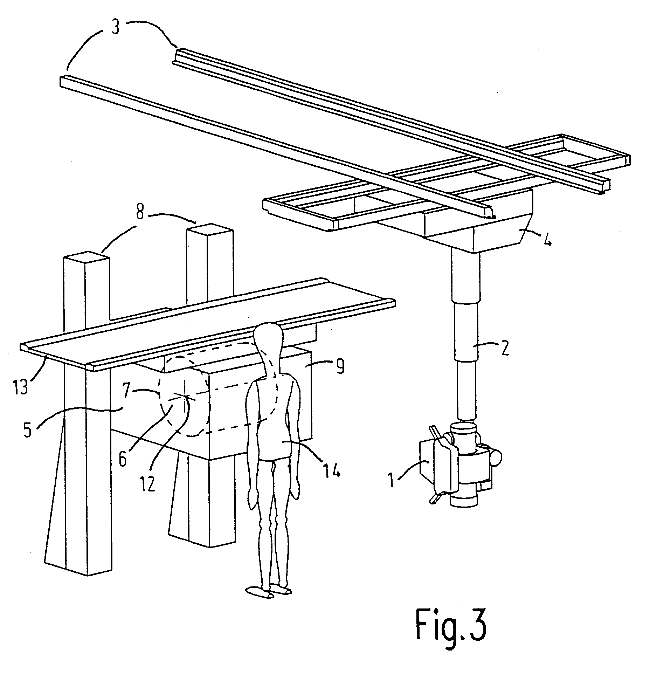

Fig. 3 zeigt die Anordnung zur Röntgenaufnahme des Thorax eines vor dem Gehäuse 5 stehenden Patienten. Mit Hilfe einer nicht dargestellten Höhenverstelleinrichtung wird das Gehäuse 5 an dem Bodenstativ 8 entsprechend der Größe des Patienten 14 in die richtige Höhe gebracht. Vorteilhafterweise stellt eine nicht dargestellte Steuereinrichtung den Röntgenstrahler 1 bei gewünschter horizontaler Strahlungsrichtung automatisch auf die gleiche Höhe ein. Möglich sind bei dieser Anordnung des Röntgenstrahlers 1 auch Röntgenaufnahmen anderer Körperteile wie beispielsweise des Kopfes oder des Knies, wozu das Gehäuse 5 und der Röntgenstrahler 1 auf die entsprechende Höhe eingestellt werden können.

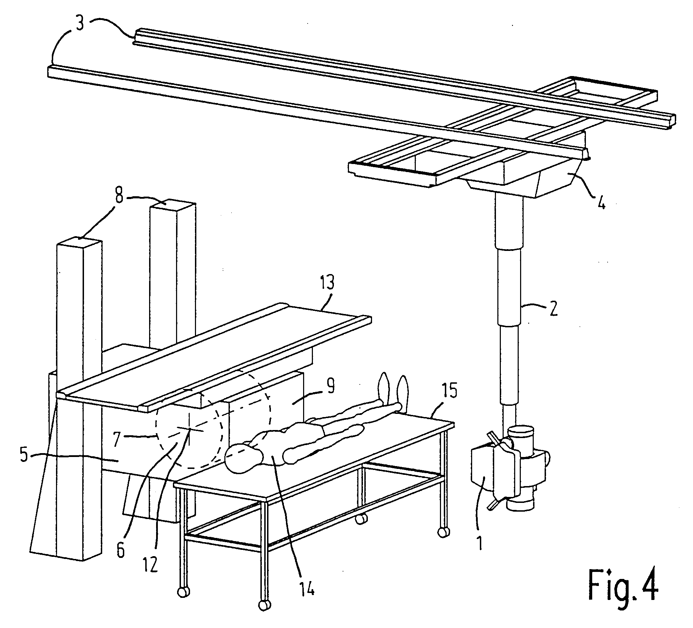

Bei gleicher Strahlungsrichtung können wie in Fig. 4 dargestellt auch Röntgenaufnahmen gemacht werden, bei denen der Patient 14 durch Zusatzhilfsmittel - im gezeigten Fall einen Tisch 15 - in die richtige Stellung zur Aufnahme gebracht wird. Andere, nicht dargestellte Beispiele für ein solches Zusatzhilfsmittel sind ein Stuhl oder ein Podest zur Aufnahme beispielsweise eines Fußes.

In Fig. 5 ist als Ausschnitt ein Teilquerschnitt des Gehäuses 5 mit dem zylinderförmigen Träger 6 gezeigt, woraus die Lage von Aufladeeinrichtung 16, Ausleseeinheit 17 und zweier Sensoreinrichtungen 18 und 19 erkennbar ist. Bei einer speziellen Ausführung mit zwei für Röntgenstrahlung transparenten Fenstern 9 und 10 sind zwei Sensoreinrichtungen 18 und 19 im Strahlenbündel zwischen den Fenstern 9 und 10 und dem Photoleiter 7 vorgesehen. Bei einer Röntgenaufnahme beispielsweise senkrecht von oben mit vertikaler Strahlungsrichtung wird die Sensoreinrichtung 18 während der Belichtung aktiviert. Diese mißt die Stärke der Ladung auf der Oberfläche des Photoleiters 7 und mit Hilfe einer Steuereinrichtung wird bei ausreichender Belichtung die Röntgenstrahlung abgeschaltet, um den Patienten nicht länger als notwendig der Röntgenstrahlung auszusetzen. Die Aufladeeinrichtung 16 und die Ausleseeinheit 17 sind dort angeordnet, wo sie nicht im Strahlengang zwischen dem Röntgenstrahler 1 und dem Photoleiter 7 liegen. Im gezeigten Fall ist dies seitlich neben dem Träger 6 im unteren, den Fenstern 9 und 10 abgewandten Bereich.

## Patentansprüche

1. Anordnung zur Erzeugung von Röntgenaufnahmen mit einem Röntgenstrahler zur Erzeugung eines Röntgenstrahlenbündels, einem Photoleiter zur Umsetzung von Röntgenstrahlung in ein Ladungsmuster, der auf einem rotationssymmetrisch ausgebildeten drehbaren Träger aufgebracht ist, einer Aufladeeinrichtung zur homogenen Aufladung der Oberfläche des rotierenden Photoleiters vor der Röntgenaufnahme, einer Ausleseeinheit, um nach einer Röntgenaufnahme das Ladungsmuster auf der Oberfläche des rotierenden Photoleiters in elektrische Bildwerte umzusetzen und einem Gehäuse zur Lagerung der rotierenden Teile, das einen für Röntgenstrahlung transparenten Bereich aufweist,
**dadurch gekennzeichnet**, daß der Röntgenstrahler in einem Verstellwinkelbereich um die Drehachse in verschiedenen Winkelstellungen positionierbar ist und daß sich der für Röntgenstrahlung transparente Bereich über einen solchen Bereich des Gehäuses erstreckt, daß eine Belichtung des Photoleiters aus jeder der im Verstellwinkelbereich einstellbaren Winkelpositionen des Röntgenstrahlers möglich ist.

2. Anordnung nach Anspruch 1,
**dadurch gekennzeichnet**, daß die Drehachse des rotationssymmetrisch ausgebildeten Trägers in horizontaler Richtung verläuft.

3. Anordnung nach Anspruch 1 oder 2,
**dadurch gekennzeichnet**, daß der Verstellwinkelbereich mindestens 90° beträgt.

4. Anordnung nach Anspruch 2,
**dadurch gekennzeichnet**, daß sich der Verstellwinkelbereich von der Senkrechten durch die Drehachse bis zur Horizontalen durch die Drehachse erstreckt.

5. Anordnung nach einem der vorhergehenden Ansprüche,
**dadurch gekennzeichnet**, daß die Aufladevorrichtung und die Ausleseeinheit nebeneinander in einem Winkelbereich zum Träger angeordnet sind, welcher außerhalb des für Röntgenstrahlung transparenten Bereichs liegt.

6. Anordnung nach einem der vorhergehenden Ansprüche,
**dadurch gekennzeichnet**, daß das Gehäuse zueinander im wesentlichen rechtwinklig angeordnete Wandungen aufweist, von denen zwei einander benachbarte Wandungen als für Röntgenstrahlung transparente Fenster ausgebildet sind.

7. Anordnung nach einem der vorhergehenden Ansprüche,
**dadurch gekennzeichnet**, daß mindestens zwei Sensoreinrichtungen in unterschiedlichen Winkelpositionen zur Erfassung der Stärke der Ladung auf der Oberfläche des Photoleiters und daß eine Steuerung zur automatischen Abschaltung der Röntgenstrahlung bei einem Grenzwert der Ladungsstärke vorgesehen ist.

8. Anordnung nach Anspruch 7,
**dadurch gekennzeichnet**, daß eine Steuerung dazu vorgesehen ist, die im Strahlengang liegende Sensoreinrichtung abhängig von der Winkelposition des Röntgenstrahlers zu aktivieren.

9. Anordnung nach einem der vorhergehenden Ansprüche,
**dadurch gekennzeichnet**, daß Mittel zur Höhenverstellung des Gehäuses vorgesehen sind.

10. Anordnung nach Anspruch 9,
**dadurch gekennzeichnet**, daß eine Steuereinrichtung dazu vorgesehen ist, abhängig von der Höhe des Gehäuses und der gewünschten Strahlungsrichtung automatisch den Röntgenstrahler in die zugeordnete Position zu bringen.
